(19) Europäisches Patentamt
European Patent Office
Office européen des brevets

(11) **EP 2 556 168 B1**

(12) **EUROPÄISCHE PATENTSCHRIFT**

(45) Veröffentlichungstag und Bekanntmachung des Hinweises auf die Patenterteilung:
**16.02.2022   Patentblatt 2022/07**

(21) Anmeldenummer: **11717207.2**

(22) Anmeldetag: **08.04.2011**

(51) Internationale Patentklassifikation (IPC):
***C12Q 1/6818*** (2018.01)    ***C12Q 1/686*** (2018.01)

(52) Gemeinsame Patentklassifikation (CPC):
(C-Sets verfügbar)
**C12Q 1/6818; C12Q 1/686**          (Forts.)

(86) Internationale Anmeldenummer:
**PCT/EP2011/055515**

(87) Internationale Veröffentlichungsnummer:
**WO 2011/124684 (13.10.2011 Gazette 2011/41)**

(54) **VERFAHREN ZUM NACHWEIS SPEZIFISCHER NUKLEINSÄURESEQUENZEN**

METHOD FOR DETECTING SPECIFIC NUCLEIC ACID SEQUENCES

PROCÉDÉ DE DÉTECTION DE SÉQUENCES D'ACIDE NUCLÉIQUE SPÉCIFIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.04.2010   DE 102010003781**

(43) Veröffentlichungstag der Anmeldung:
**13.02.2013   Patentblatt 2013/07**

(73) Patentinhaber: **IST Innuscreen GmbH
13125 Berlin (DE)**

(72) Erfinder:
• **GRASER, Elmara**
  **13086 Berlin (DE)**
• **HILLEBRAND, Timo**
  **15366 Hoppegarten (DE)**

(74) Vertreter: **Wehlan, Martin
Patentanwälte Wehlan & Wehlan
Möllendorffstraße 49
10367 Berlin (DE)**

(56) Entgegenhaltungen:
**EP-A1- 0 566 751          EP-B1- 0 826 066
WO-A1-2008/104791      WO-A2-2006/041524
WO-A2-2009/000764      DE-A1- 19 811 731
US-B1- 6 174 670**

(52) Gemeinsame Patentklassifikation (CPC): (Forts.)

C-Sets
**C12Q 1/6818, C12Q 2565/101, C12Q 2563/131,
C12Q 2527/107;
C12Q 1/686, C12Q 2565/101, C12Q 2563/131,
C12Q 2527/107**

C-Sets
**C12Q 1/6818, C12Q 2565/101, C12Q 2563/131,
C12Q 2527/107;**

## Beschreibung

Die vorliegende Erfindung betrifft ein Verfahren und einen Testkit zum Nachweis spezifischer Nukleinsäuresequenzen, umfassend die Schritte 1. matrizenabhängige Neusynthese der Targetnukleinsäure, 2. targetspezifische Sondenhybridisierung und 3. Nachweis des Hybridisierungsereignisses. Dabei erfolgt der Nachweis der Hybridisierungsreaktion fluorometrisch in Form eines FRET-Paares. Zusätzlich kann eine Verifizierung des Ergebnisses nachfolgend immunologisch erfolgen. Die Detektionsreaktion erfolgt immer zeitlich nach erfolgter matrizenabhängiger Neusynthese.

## Stand der Technik

Die Gendiagnostik ist zu einem unverzichtbaren Bestandteil der modernen medizinischen Labordiagnostik, der forensischen Diagnostik, der veterinärmedizinischen Labordiagnostik oder der Lebensmittel- und Umweltdiagnostik geworden.

Revolutioniert wurde die genetische Diagnostik mit der Erfindung der PCR-Technologie, die es gestattet, jede beliebige Nukleinsäuresequenz spezifisch zu vervielfachen.

Unter Verwendung der PCR existiert eine Vielzahl von Methoden, die in Kombination mit der PCR-Technologie darüber hinaus den spezifischen Nachweis einer erfolgten Amplifikation ermöglichen. Insbesondere die Vorgaben einer exakten genetischen Diagnostik müssen sich Verfahren bedienen, die sicherstellen, dass ein erzeugtes Amplifikationsprodukt auch der spezifisch nachzuweisenden Zielsequenz entspricht. Die weit verbreitete Nutzung der Visualisierung eines PCR-Produktes mittels Gelelektrophorese ist dazu nicht ausreichend.

Eine prinzipiell sehr schnelle und ohne großen experimentellen Aufwand realisierbare Möglichkeit zum Nachweis spezifischer Nukleinsäuren kann durch die sog. RealTime-PCR-Methoden erreicht werden. Hierbei ist die Amplifikationsreaktion mit der eigentlichen Nachweisreaktion direkt gekoppelt.

Eine weit verbreitete Methode zum Nachweis spezifischer Nukleinsäuren ist die Light-Cycler-Technologie (Fa. Roche). Die Firma Roche entwickelte dazu spezielle Hybridisierungssonden, bestehend aus zwei verschiedenen Oligonukleotiden, die jeweils mit nur einem Fluorochrom markiert sind. Am 3'-Ende der einen Sonde befindet sich der Akzeptor, das andere Oligonukleotid ist am 5'-Ende mit einem Donor versehen. Die Sonden werden so gewählt, dass sie beide an den gleichen DNA-Strang binden, wobei der Abstand zwischen Akzeptor und Donor nur maximal 1 bis 5 Nukleotide betragen darf, damit es zum sog. FRET-Effekt kommen kann. Die Messung der Fluoreszenz erfolgt während des Annealingschrittes, wobei nur Licht dieser Wellenlänge nachweisbar ist, solange beide Sonden an die DNA gebunden sind. Der Schmelzpunkt beider Sonden sollte bei diesem System identisch sein. Durch die Verwendung zweier hybridisierender Sonden zusätzlich zu den verwendeten Primern ist die Spezifität dieses Detektionssystems äußerst hoch.

Eine weitere RealTime-PCR-Anwendung zum Nachweis spezifischer Nukleinsäure-Targets kann mit sog. Double-Dye-Sonden, die in den Patentschriften US 5210015 und US 5487972 offenbart wurden (TaqMan- Sonden), durchgeführt werden. Double-Dye-Sonden tragen zwei Fluorochrome auf einer Sonde. Der Reporterfarbstoff befindet sich hier am 5'-Ende, der Quencherfarbstoff am 3'-Ende. Zusätzlich befindet sich am 3'-Ende der Sonde eventuell noch eine Phosphatgruppe, damit die Sonde bei der Elongation nicht als Primer fungieren kann. Solange die Sonde intakt ist, ist die freigesetzte Lichtstärke gering, da fast die gesamte Lichtenergie, die nach der Anregung des Reporters entsteht, aufgrund der räumlichen Nähe vom Quencher aufgenommen und umgeformt wird. Das emittierte Licht des Reporterfarbstoffes wird "gequenched", d.h. gelöscht. Dieser FRET-Effekt bleibt auch erhalten, nachdem die Sonde an den komplementären DNA-Strang gebunden hat. Während der Elongationsphase trifft die Polymerase auf die Sonde und hydrolysiert sie. Man bezeichnet die Fähigkeit der Polymerase, ein Oligonukleotid (bzw. eine Sonde) während der Strangsynthese zu hydrolysieren, als 5'-3' Exonukleaseaktivität. Nicht alle Polymerasen haben eine 5'-3' Exonukleaseaktivität (Taq- und Tth-Polymerase). Als erstes wurde dieses Prinzip für die Taq-Polymerase beschrieben. Das Prinzip wird als TaqMan-Prinzip bezeichnet. Nach Sondenhydrolyse befindet sich der Reporterfarbstoff nicht mehr in räumlicher Nähe zum Quencher. Die emittierte Fluoreszenz wird jetzt nicht mehr umgeformt, dieser Fluoreszenzanstieg wird gemessen.

Es gibt auch weitere, FRET-Effekt basierte Verfahren, die sowohl Abnahme als auch die Zunahme der Probenfluoreszenz detektieren.

In der Druckschrift (JP002001029072AA) werden Reporter und Quencher an die einzelnen der Reaktion beigefügte dNTP's (Desoxyribonukleotide) gekoppelt. Beim Einbauen dieser Nukleotide in die amplifizierte Nukleinsäure wird die Fluoreszenz der Probe durch das FRET-Effekt vermindert. Nachteil der Methode ist der Einbau der markierten Nukleotide auch bei einem Mispriming und bei den Primerdimeren. Damit kann ein solches Verfahren nicht für diagnostische Anwendungen eingesetzt werden.

Weitere Druckschriften betreffen Verfahren, bei denen die FRET-Paarmarkierungen nicht an einem Oligonukleotid angebracht sind, sondern sich auf mehrere Primer- bzw. Sondenmoleküle verteilen.

Bei der Offenlegungsschrift WO 2009/126678 A2 bilden zwei Hybridisierungssonden eine Hairpin-Formation, durch die Reporter und Quencher räumlich zusammenkommen.

Bei der Offenlegungsschrift DE 10250948 A1 sind die jeweils markierten Sonden miteinander hybridisiert. Bei Vorhandensein einer Targetnukleinsäure kann eine der Sonden einen Duplex mit der Targetnukleinsäure bilden, wodurch die FRET-Wechselwirkung aufgehoben wird.

Bei der Offenlegungsschrift DE 102005000021 A1 bilden zwei markierte Sonden einen Triplex mit der Target-Nukleinsäure, durch die Exonukleaseaktivität der Taq-Polymerase wird die Fluoreszenz freigesetzt.

Bei der Patentschrift EP1384789 B1 und einer im Patent zitierten Publikation (Bernard, P.S., et al. Anal Biochem 255 (1998) 101-7) wird jeweils ein markierter Primer und eine markierte Sonde zu einer Real-Time-Beobachtung der Amplifikation benutzt.

Eine weitere Möglichkeit zum spezifischen Nachweis von Amplifikationsprodukten mittels RealTime-PCR-Technologie besteht in der Nutzung von interkalierenden Farbstoffen (Ethidiumbromid, Hoechst 33258, Yo-Pro-1 oder SYBR Green™ u.ä.). Diese Farbstoffe emittieren nach Anregung durch energiereiches UV-Licht Licht im sichtbaren energieärmeren Wellenlängenbereich (Fluoreszenz). Liegt der Farbstoff als freier Farbstoff im Reaktionsgemisch vor, ist die Emission sehr gering. Erst durch die Interkalierung des Farbstoffs, d.h. durch die Einlagerung in die kleine Furche doppelsträngiger DNA-Moleküle wird die Lichtemission stark verstärkt. Die Farbstoffe sind preiswert und universell verwendbar, da mit ihnen prinzipiell jede PCR-Reaktion in Echtzeit verfolgt werden kann. Außerdem haben sie eine hohe Signalstärke, da jedes DNA-Molekül mehrere Farbstoffmoleküle bindet. Aus den Vorteilen resultiert aber auch ein extremer applikativer Nachteil: Durch interkalierende Farbstoffe kann man prinzipiell nicht zwischen korrektem Produkt und Amplifikations-Artefakten (wie z.B. PrimerDimeren oder Fehlprodukten) unterscheiden. Entstehende Primer-Dimere und andere Artefakte binden naturgemäß ebenfalls interkalierende Farbstoffe und führen somit zu einem unspezifischen Anstieg der Fluoreszenz auch in negativen Proben. Eine klare Differenzierung zwischen spezifischem Amplifikationsereignis bzw. Artefakt ist aber zwingend notwendig. Um dies dennoch zu erreichen, nutzt man eine sog. Schmelzpunktanalyse am Ende der eigentlichen PCR-Reaktion. Das Reaktionsgemisch wird dabei in 1-Grad-Schritten von 50 °C auf 95 °C erhitzt. Bei diesem Prozess wird kontinuierlich die Fluoreszenz gemessen. Der Punkt, an dem doppelsträngige DNA schmilzt, ist durch einen Abfall (Peak) der Fluoreszenz des interkalierenden Farbstoffes gekennzeichnet, da der interkalierende Farbstoff von der einzelsträngigen DNA dissoziiert. Wenn die PCR optimal eingestellt ist, sollte ein Schmelzpunktpeak zu erwarten sein, der spitz zuläuft. Dieser Schmelzpunkt stellt das spezifische, zu erwartende Produkt dar. Unterschiedlich große Produkte und Produkte anderer Sequenz haben unterschiedliche Schmelzpunkte.

Mittels RealTime-PCR-Anwendungen ist es darüber hinaus auch möglich, eine Quantifizierung der nachzuweisenden Targets durchzuführen.

Wie schon aufgeführt, erfüllen die beschriebenen Verfahren die Notwendigkeit des spezifischen Nachweises eines Amplifikationsproduktes.

Ein großer Nachteil besteht allerdings darin, dass sie auf sehr teuren Geräteplattformen implementiert sind, welche den Prozess sowohl der Amplifikation als auch nachfolgenden, der Fragestellung entsprechenden optischen Detektion in einer Hardware- Lösung vereinen müssen. Weiterhin basieren viele dieser beschriebenen Nachweisverfahren immer auf der Echtzeitverfolgung des Amplifikationsprozesses. Auf dieser Strategie basierend, erfolgt auch die Aufarbeitung der gemessenen Fluoreszenzwerte im Verlauf der Amplifikationsreaktion. Dem Fachmann ist klar, dass damit verbunden auch ein enorm hoher Grad an Analysealgorithmen in RealTime-Systeme integriert sein muss. Dies erklärt letztlich den hohen finanziellen Aufwand, der für die Nutzung von RealTime-PCR-Systemen investiert werden muss. Letztlich ist auch die Bedienung solcher Gerätesysteme an ein hohes Maß an Expertise gebunden.

Neben den dargestellten RealTime-PCR-basierten diagnostischen Nachweisen existieren aber auch alternative Varianten zum spezifischen Nachweis von Nukleinsäuren.

Preiswertere Verfahren zum Nachweis von Nukleinsäuren sind in diesem Zusammenhang z.B. PCR-ELISA. Bei dieser Methode wird die zu untersuchende DNA-Sequenz amplifiziert und das erzeugte DNA-Fragment nachfolgend an einer festen Phase (z.B. Mikrotiterplatte oder Streifen) kovalent immobilisiert, nachfolgend zu einem Einzelstrang denaturiert und mit einer sequenzspezifischen Sonde hybridisiert. Die erfolgreiche Anbindung der Sonde kann durch eine antikörpervermittelte Farbreaktion sichtbar gemacht werden. Eine andere Variante basiert darauf, dass man die Sonden an eine feste Phase immobilisiert und nach erfolgter Denaturierung des PCR-Produktes dieses in Kontakt mit der immobilisierten Sonde bringt. Der Nachweis eines erfolgten Hybridisierungsereignisses erfolgt in Analogie zur ersten Verfahrensvariante.

PCR-ELISA sind prinzipiell einfach in der Durchführung, umfassen allerdings multiple Verfahrensschritte, so dass neben der benötigten Zeit zur Durchführung der PCR auch mehrere Stunden Arbeitszeit für die Durchführung des nachfolgenden Detektionsverfahrens benötigt werden. Ein solches Verfahren benötigt in der Regel 8h und ist damit auch als Schnelltest nicht geeignet.

Darüber hinaus werden auch einige Geräte wie eine Temperierstation, ein sog. Washer oder auch ein Messgerät zum Nachweis des Hybridisierungssignals benötigt. Zusätzlich sind ggf. weitere Spezialgräte oder Spezialverbrauchsmaterialien notwendig.

Weitere einfache Verfahren zum Nachweis von Amplifikationsprodukten basieren auf der Amplifikation der Zielsequenzen und der nachfolgenden Hybridisierung von Amplifikationsprodukten an einer Membran. Auch bei diesem Verfahren gibt es dem Fachmann bekannte verschiedene Varianten. Diese Verfahren sind aber auch wieder aufwendig in der Durchführung, benötigen eine Vielzahl an abzuarbeitenden Verfahrensschritten und sind damit als Schnelltests nicht geeignet. Dies betrifft dann auch die Nutzung von Biochip-Strategien, welche die Hybridisierung von PCR-Produkten mit Hybridisierungssonden zum Nachweis der Spezifität nutzen. Auch diese Verfahren sind aufwendig und an sehr teure Geräteplattformen gebunden.

Eine deutliche Reduktion von Arbeitsschritten wird in der Druckschrift KR 1020060099022 A (Method and kit for rapid and Accurate detection and analysis of nucleotide sequence with naked eye by using membrane lateral flow analysis) offenbart.

Hierbei wird ein sog. Lateral-Flow-Verfahren genutzt, um Nukleinsäuren zu detektieren. Auch dieses Verfahren bedient sich der Technologie der Hybridisierung von Nukleinsäuren an einer festen Phase. Vorteilhaft ist, dass es sich bei einem Lateral-Flow-Verfahren um eine kleines, handliches Testformat handelt (Streifentest).

Eine sehr schnelle Nachweismethodik, welche sich auch dem Nachweis von Amplifikationsprodukten mittels eines Teststreifens bedient und kommerziell angeboten wird, basiert im Gegensatz zur obigen Druckschrift wiederum auf einem völlig anderen Prinzip. Hierbei erfolgt die Durchführung der PCR-Reaktion mit einem biotinylierten Primer und einem nicht-biotinylierten Primer. Nach Durchführung der PCR liegt ein PCR-Produkt vor, welches an einem Ende damit Biotin-markiert ist. Der Nachweis nutzt einen Teststreifen (z.B. Fa. Millenia, Amodia etc.), der zwei getrennte Bindungsstellen enthält. Eine Streptavidin-Stelle zur Kopplung des Biotin-markierten DNA-Stranges und eine FITC-Bindungsstelle zur Funktionskontrolle des Teststreifens.

Der Nachweis des PCR-Produktes realisiert sich dadurch, dass man nach durchgeführter PCR den PCR-Ansatz denaturiert und mit einer zum Biotin-markierten DNA-Strang komplementären Sonde hybridisiert. Die Sonde ist FITC-markiert.

Zum Nachweis wird der PCR-Hybridisierungs-Ansatz mit einem Laufpuffer gemischt und auf den Teststreifen aufgetragen. Nach der Testbeschreibung bindet sich der biotinylierte DNA-Strang an die Streptavidin-Bindungsstelle des Streifens. Der Nachweis erfolgt über die FITC-Markierung der mit dem DNA- Strang hybridisierten Sonde. Es bildet sich ein typisches Signal in Form eines Streifens aus. Dieses Signal soll der spezifische Nachweis des Amplifikationsproduktes sein. Das Verfahren kombiniert aber nicht die Hybridisierung der Sonde mit dem Prozess der PCR, sondern führt dies als separaten Verfahrensschritt durch.

Die Offenlegungsschrift WO 2009/000764 A2 erlaubt die Durchführung einer Amplifizierung und Hybridisierung des PCR-Produktes in einem Reaktionsansatz. Dabei entsteht ein doppelt markiertes Amplifikat-Sonden-Dimer, das nachträglich z.B. mittels eines Lateral-Flow-Streifens visualisiert werden kann. Dies ist eine sehr preiswerte und vor allem geräteunabhängige Möglichkeit, ein PCR-Hybridisierungs-Produkt zu visualisieren. Nachteil dieses Verfahrens ist aber, dass es sich primär nicht um einen homogenen Assay handelt, da nach erfolgter Amplifizierungs-/ Hybridisierungsreaktion des Reaktionsgefäß geöffnet werden muss, um das Amplifikationsgemisch auf einen Lateral-Flow-Streifen überführen zu können.

Damit erfolgt die eigentliche Detektionsreaktion außerhalb der Reaktionskavität, in welcher die Amplifizierungs-/Hybridisierungsreaktion durchgeführt wird.

Das Verfahren erlaubt auch keine numerische Darstellung des Detektionsergebnisses, sondern begrenzt sich immer nur auf eine visuell zu befundene JA-NEIN-Aussage. Auch eine Quantifizierung der Reaktionsprodukte ist auf einem Lateral-Flow-Streifen nicht gegeben, da die obere Grenze durch die Bindungskapazität des Streifens vorgegeben wird.

In der internationalen Patentanmeldung WO 2005/51967 A2 werden markierte Oligonukleotide mit mehreren Fluorophoren beschrieben. Das Verfahren zum Trennen der Fluorophore, einschließlich der Spaltung der markierten Oligonukleotide, verwendet Enzyme mit 5'-Exonukleaseaktivität.

Gegenstand der Druckschrift WO 03/072051 A2 ist eine Fluoreszenz-Energietransfer (FET) -markierte Sonde mit einem Nukleinsäure-Interkalator, der eine polycyclische Verbindung enthält, die an ein FET-markiertes Oligonukleotid gebunden ist, wobei der Nukleinsäure-Interkalator kovalent an das 3'-Ende des FET-markierten Oligonukleotids gebunden ist, und wobei das FET-markierte Oligonukleotid einen DarkQuencher darstellt, der an dessen 3'-Ende positioniert ist und wobei die FET-markierte Sonde 3'-5'-Exonuklease-resistent ist.

Die Veröffentlichung von E Lyon et al., "LightCycler Technology in Molecular Diagnostics", J. Mol. Diagn (2009) 11 (2) 93-101 berichtet über eine PCR mit Realtime-Fluorescent-Monitorung und Schmelzkurven-Analyse. Dabei ist die Durchführung der PCR innerhalb von 15 Minuten möglich. Das Review beschreibt die bedeutenden Fortschritte der LightCycler-Technologie innerhalb der letzten 15 Jahre.

Ziel der vorliegenden Erfindung bestand darin, ein universell nutzbares Verfahren zum spezifischen Nachweis von Targetnukleinsäuren bereitzustellen, welches es ermöglicht, die Nachweisreaktion auch in Form eines homogenen Assays durchführen zu können, d.h. dass die Detektion der Nachweisreaktion schon in der Reaktionskavität erfolgt, in der die eigentliche Amplifizierungs-/Hybridisierungsreaktion auch abläuft. Ziel war auch die diagnostische Sicherheit einer Nachweisreaktion durch eine quasi zusätzliche Doppeldetektion zu ermöglichen, da eine Reihe von Testdurchführungen neben einer ersten Nachweisreaktion (z.B. RealTime-PCR) einen zweiten Kontrollnachweis benötigen (z.B.

Auftragen der Amplifikationsprodukte auf ein Agarosegel). In Erweiterung der Zielstellung könnte ein solches neuartiges Verfahren auch in der Form eingesetzt werden, dass die Nachweisreaktion als homogener Assay erfolgt (geräteassoziiert) oder aber auch alternativ dazu als ein Lateral-Flow-Streifen-basierter Test (geräteunabhängig).

Ein Verfahren mit einer ähnlichen Zielstellung wurde von Piepenburg et al. (PLOS biologie, july 2006, Volume 4, Issue 7, 1115-1121) publiziert. Hierbei wird eine Rekombinase-gekoppelte isotermale PCR durchgeführt. Die doppelmarkierte Sonde weist eine integrierte Schnittstelle für eine Nuklease vor. Bei der Hybridisierung der Sonde wird der gebildete Doppelstrang von der Nuklease geschnitten und dadurch die Fluoreszenz freigesetzt. Nach der Zerschneidung mit der Nuklease kann die markierte Sonde als Primer fungieren. Zusammen mit dem anderen markierten Primer entsteht dann ein doppelmarkiertes PCR-Produkt, das auf einem LFA-Streifen nachgewiesen werden kann. Nachteilig ist, dass dieses Verfahren chemisch sehr komplex und schwierig ist. Darüber hinaus muss schon vor Beginn der Versuchsdurchführung entschieden werden, welche der Nachweismethoden und daher welche Markierungen bevorzugt werden. Würde eine solche Entscheidung nicht getroffen werden, dann muss die Sonde an mindestens vier Stellen präpariert werden: 1. Reporter Fluorophor, 2. Quencher, 3. Schnittstelle der Nuklease, 4. Amplifikationsblockade am 3'-Ende.

Die bestehende Aufgabenstellung wurde gemäß den Merkmalen der Patentansprüche überraschend einfach gelöst. Das erfindungsgemäße Verfahren kombiniert eine matrizenabhängige DNA-Neusynthese mit einem Hybridisierungsschritt. Die erfindungsgemäße Auswahl der Markierungen der an den Reaktionen beteiligten Oligonukleotide erlaubt sowohl eine reaktionsabhängige Fluoreszenzmessung, wobei die Markierungen als ein FRET-Paar fungieren und der Nachweis in Folge des FRET-Effekts mittels einer Endpunktdetektion erfolgt Damit verbundenen ist eine numerische und ggf. quantitative Auswertung. Zusätzlichkann auch eine geräteunabhängige Visualisierung der Reaktion z.B. auf einem Lateral-Flow-Streifen erfolgen. Ein solches Messprinzip kann gerätetechnisch damit auch auf die Verwendung von teuren Real-Time-Gerätesystemen verzichten.

Das erfindungsgemäße Verfahren basiert dabei auf folgenden Schritten:

### A. Bereitstellung eines Reaktionsansatzes, bestehend aus:

♦ einer eine Nukleinsäure enthaltenden Probe, in der die Target-Nukleinsäure nachgewiesen werden soll
♦ mindestens ein durch eine Markierung 1 markiertes Oligonukleotid, das vollständig oder teilweise zu der Targetsequenz komplementär ist und als ein Primer in einer matrizenabhängige Neusynthese der Targetnukleinsäure fungiert (Oligo Typ 1)
♦ mindestens ein durch eine Markierung 2 markiertes Oligonukleotid, das aufgrund seiner niedrigeren Schmelztemperatur als Oligonukleotid 1 nicht am DNA-Neusyntheseprozess beteiligt ist, aber mit dem DNA-Neusyntheseprodukt von Oligonukleotid 1 teilweise oder vollständig hybridisieren kann (Oligo Typ 2)
♦ ein Chemikalien-/Enzym-Gemisch ggf. auch mit weiteren, nicht markierten Oligonukleotiden, das eine matrizenabhängige Neusynthese der Targetnukleinsäure ermöglicht.

Der Begriff "teilweise komplementär" bedeutet im Sinne der Erfindung, dass eine ausreichende Komplementarität vorhanden sein muss. im vorliegenden Fall müssen mindestens 50 %, vorzugsweise 70 % des markierten Oligonukleotids zur Targetnukleinsäure komplementär sein.

Der Begriff "matrizenabhängig" bedeutet im Sinne der Erfindung, dass die Neusynthese der Targetnukleinsäure durch die verwendeten Primer gesteuert wird.

Erfindungsgemäß werden die Markierungen der beiden Oligonukleotide (Oligo Typ 1 und Oligo Typ 2) so ausgewählt, dass sie zusammen ein FRET-Paar bilden (z.B. FITC/TAMRA, FAM/TAMRA, FAM/BHQ1 u.ä.) und in Bezug auf die erfindungsgemäße Doppeldetektion auch komplementäre Bindungspartner auf einem Lateral-Flow-Streifen haben können.

### B. Durchführung der matrizenabhängigen DNA-Neusynthese mit integrierter Sondenhybridisierung

In der Abhängigkeit von der Art der Target-Nukleinsäure wird entweder eine Reverse Transkriptase Reaktion (bei RNA, die in einer sehr großen Kopienzahl vorkommt z.B. rRNA, tmRNA) oder eine Amplifikation (bei DNA) oder auch die beiden Reaktionen nacheinander folgend (bei einer raren RNA z.B. mRNA, Proben mit einer geringen Partikelzahl) durchgeführt.

In dieser ersten Reaktion ist auf Grund des erfindungsgemäßen Verfahrens nur das Oligo Typ 1 beteiligt. Das Oligo Typ 1 fungiert als ein Primer entweder in einer RNA-abhängigen Reversen Transkription (dadurch entsteht ein markierter cDNA-Strang) oder in einer Amplifikation der Target-DNA bzw. cDNA (dadurch entsteht ein markiertes PCR-Produkt). OneStep RT-PCR kann gleichfalls durchgeführt werden. Dabei erhöht ein zweites, nichtmarkiertes Primer-Oligonukleotid die Ausbeute der PCR- Reaktion.

Das Oligo Typ 2 ist auf Grund seiner niedrigeren Anealingtemperatur gemäß dem erfindungsgemäßen Verfahrens nicht an der DNA-Neusynthese beteiligt. Nachfolgend wird der Reaktionsansatz erhitzt bei einer Temperatur > 90°C. Dieser Schritt führt zur thermischen Strangtrennung. Nach dem Ablauf dieser thermischen Denaturierungsreaktion wird der Reaktionsansatz auf die Hybridisierungstemperatur des Oligos Typ 2 abgekühlt. Während dieses Schrittes bindet das Oligo Typ 2 spezifisch an den komplementären DNA-Strang. Dieser Strang trägt dabei die Markierung 1, welche durch das Oligo Typ 1 in das Reaktions-Produkt eingebaut wurde.

**C. Nachweis des Hybridisierungsereignisses**

Die Nachweisreaktion kann in zwei Varianten erfolgen, erfindungsgemäß mittels einer Fluoreszenzmessung. Zusätzlich können aber auch beide Nachweisvarianten parallel eingesetzt werden, wobei die Nachweisreaktion außerhalb des Reaktionsgefäßes an einer festen Phase zusätzlich durchgeführt werden kann.

1. Nachweis der Hybridisierungsreaktion mittels einer Fluoreszenzmessung.

Die durch die von den beiden Oligos Typ 1 und Typ 2 eingebauten Markierungen bilden ein FRET-Paar. Die im erfindungsgemäßen Verfahren erfolgende Hybridisierung des Oligos Typ 2 an das Syntheseprodukt des Oligos Typ 1 führt zu einem FRET-Effekt zwischen der Markierung 1 und 2. Dieser Effekt führt jetzt zu einer messbaren Verringerung der Fluoreszenz. Diese Reduzierung der Fluoreszenz wird numerisch ausgewertet und ermöglicht so den eindeutigen Nachweis der Reaktion.

2. Nachweis der Hybridisierungsreaktion außerhalb oder innerhalb des Reaktionsgefäßes an einer festen Phase dadurch gekennzeichnet, dass:

Die feste Phase (z.B. ein Lateral-Flow-Streifen, Mikrotiterplatte, Mikropartikel) enthält eine Bindungsstelle für eine der Markierungen der Oligos Typ1 oder Typ 2 und / oder Antikörper oder andere Bindungsmoleküle gegen die Markierungsmoleküle der Oligos Typ1 oder Typ 2, welche an die Markierungsmoleküle Typ 1 oder Typ 2 binden können (z.B. kovalente oder Wasserstoffbrückenbindung oder über Brückenmoleküle). Darüber hinaus befindet sich auf der festen Phase ein Nachweismolekül für eine Visualisierung bzw. Messung des Hybridisierungsereignisses oder ein solches Nachweismolekül wird der Detektionsreaktion zugeführt. Das Nachweismolekül kann aber auch schon während der Amplifizierungs-/ Hybridisierungsreaktion in das nachzuweisende Hybridisierungsprodukt eingebaut worden sein.

Zusammenfassend steht mit dem erfindungsgemäßen Verfahren nun ein extrem einfaches und universell einsetzbares gendiagnostisches Nachweisverfahren zur Verfügung.

Die Detektion einer nachzuweisenden diagnostisch relevanten Targetnukleinsäure erfolgt in Form eines homogenen Assays erfindungsgemäß über die Endpunkt-Fluoreszenzmessung einer Fluoreszenzlöschung nach dem FRET-Prinzip. Das Ergebnis kann numerisch erfasst werden und erlaubt auch eine Quantifizierung der nachzuweisenden Targetnukleinsäure (unter Verwendung eines internen Standards) detektiert und quantifiziert. Darüber hinaus ermöglicht das erfindungsgemäße Verfahren auch einen hochspezifischen Doppelnachweis, da nach erfolgter Fluoreszenzdetektion nachfolgend eine Ergebnisverifizierung auf einem Lateral-Flow-Streifen durchgeführt werden kann. Eine solche Verifizierung ist damit diagnostisch sehr viel exakter, als ein bisher möglicher Nachweis von RealTime-PCR- Produkten auf einem Agarosegel. Das Verfahren erlaubt im Bedarfsfall auch die Durchführung der Tests unabhängig voneinander (Test mittels Fluoreszenzdetektion oder Test mittels dem Nachweis z.B. auf einem Lateral-Flow-Streifen).

Diese elegante neuartige Testdurchführung, insbesondere auch die kombinierte Testdurchführung (Fluoreszenznachweis und nachfolgende Verifizierung der ersten Testreaktion an einer festen Phase) werden erfindungsgemäß dadurch ermöglicht, dass während der Amplifikation/Hybridisierung die hybridisierte Sonde (Oligo Typ 2) nicht durch die Taq-Polymerase zerstört wird, sondern auch nach dem Reaktionsablauf im hybridisierten Zustand bleibt im Gegensatz zum homogenen TaqMan-Exonuklease-Assay.

Die erfindungsgemäße Integration einer Hybridisierungssonde in die Reaktion gibt die Sicherheit, dass das amplifizierte Fragment wirklich die Zielsequenz beinhaltet. Dadurch werden die durch Mispriming entstehenden falschpositiven Resultate ausgeschlossen. Die Verwendung der chemisch modifizierten Sonde (vorzugsweise Phosphorylierung des letzten Nukleotids der Sonde) verhindert die Verlängerung der Sonde durch 5'-->3'-Polymeraseaktivität und verhindert damit, dass die Sonde als Primer fungiert und unspezifische PCR-Artefakte (Primer- Dimere) erzeugt, die als falsch positive Signale detektiert werden würden.

Im Unterschied zu RealTime-PCR-Verfahren erfolgt die Detektion des spezifischen Nachweissignals nicht während der Amplifikation, wo die Fluoreszenz entweder durch die von der Taq-Polymerase durchgeführten Sondenhydrolyse freigesetzt wird (EP 0972 848 A2) oder durch den FRET-Effekt vermindert wird (EP1384789 B1), sondern erst nach dem Abschluss der Amplifizierungs-/Hybridisierungs-Reaktion. Dies bedingt auch den positiven Effekt, dass das Verfahren unabhängig von gerätetechnischer Ausrüstung ist. Messungen können sowohl in einem Real-Time-PCR-Gerät als auch nach dem Abschluss der Reaktion mit einem Fluoreszenzreader durchgeführt werden (s. Ausführungsbeispiele).

Das erfindungsgemäße Verfahren unterscheidet sich auch von der Patentschrift (EP 0 826 066 B1), welche ebenfalls eine Kombination von PCR und Hybridisierung darstellt. Auch bei diesem Verfahren wird wiederum ein durch FRET-Effekt vermitteltes Fluoreszenzsignal detektiert. Dieses entsteht während des Amplifikationsvorgangs durch die Hybridisierung einer doppeltmarkierten Sonde, die eine niedrigere Annealingtemperatur hat, als die Primer. Die Freisetzung der Fluoreszenz erfolgt dabei nicht durch Hydrolyse der Sonde in Folge der Exonukleaseaktivität der Polymerase, sondern dadurch, dass bei der Hybridisierung die sekundäre Struktur der Sonde aufgelöst wird und durch die Entfernung des Reporters von dem Quencher die Fluoreszenz freigesetzt wird. Hierbei können nur Enzyme für die Amplifikation eingesetzt werden, die keine Exonukleaseaktivität besitzen (z.B. Klenow Fragment oder T4 oder T7 Polymerasen).

Erstmals wurde ein homogenes Verfahren zur Detektion der Anwesenheit einer Zielnukleinsäure in einer Probe bereitgestellt, wobei der Reaktionsansatz enthält:

○ Probennukleinsäure, in der die Target-Nukleinsäure vermutet wird
○ mindestens ein durch eine Markierung 1 markiertes Oligonukleotid, das vollständig oder teilweise zu der Targetsequenz komplementär ist und als ein Primer in einer matrizenabhängigen Neusynthese der Targetnukleinsäure fungiert (Oligo Typ 1)
○ mindestens ein durch eine Markierung 2 markiertes Oligonukleotid, das aufgrund seiner niedrigeren Schmelztemperatur als das Oligonukleotid 1 nicht am DNA-Neusyntheseprozess beteiligt ist, aber mit dem DNA- Neusyntheseprodukt von Oligonukleotid 1 teilweise oder vollständig hybridisieren kann (Oligo Typ 2)
○ ein Chemikalien- Enzym-Gemisch ggf. auch mit weiteren, nicht markierten Oligonukleotiden, das eine matrizenabhängige Neusynthese der Targetnukleinsäure ermöglicht.

Das Verfahren umfasst folgende Schritte:

▪ matrizenabhängige Neusynthese der nachzuweisenden Targetnukleinsäure mit mindestens einem Oligo vom Typ 1 und ggf. anschließender Strangtrennung
▪ Hybridisierung des Syntheseproduktes des jeweiligen Oligos Typ 1 mit mindestens einem Oligo Typ 2
▪ Nachweis der Hybridisierungsreaktion mit Hilfe einer Fluoreszenzmessung nach dem FRET-Prinzip mittels einer Endpunktdetektion.

Die Markierungen 1 und 2 bilden ein FRET-Paar. Die Hybridisierung des Oligos Typ 2 an das Syntheseprodukt des Oligos Typ 1 führt zu einer durch das FRET- Effekt zwischen der Markierung 1 und 2 bedingten, messbaren Verringerung der Fluoreszenz.

Der Nachweis der Hybridisierungsreaktion ist auch außerhalb des Reaktionsgefäßes möglich. Im Fall des Nachweises an einer festen Phase enthält die feste Phase eine Bindungsstelle für eine der Markierungen des Oligos Typ1 oder Typ2 und/ oder Antikörper oder andere Bindungsmoleküle gegen die Markierungsmoleküle des Oligos Typ1 oder Typ 2, die an die Markierungsmoleküle Typ 1 oder Typ2 binden (z.B. kovalente oder Wasserstoffbrückenbindung oder über Brückenmoleküle) und gleichzeitig ein Nachweismolekül für eine Visualisierung bzw. Messung des Hybridisierungsereignisses, bzw. wird der an der festen Phase gebundenen Probe solches Molekül zugefügt.

Die Oligos Typ 1 und Typ 2 können auch andere Markierungen als die im Anspruch 4 genannten Markierungen tragen. Die zusätzlichen Markierungen können zum Nachweis des Hybridisierungsereignisses an der festen Phase (1e) benutzt werden.

Es ist auch möglich, anstelle der Standard-Amplifikation eine asymmetrische Amplifikation durchzuführen.

Gemäß einer bevorzugten Ausführungsform der Erfindung ist die Schmelztemperatur ($T_m$) des Oligos Typ 1 vorzugsweise 5°C bis 15°C höher ist als die $T_m$ des Oligos Typ 2. Nach der Hybridisierung sind die Markierungen 1 und 2 vorzugsweise 1 bis 50 bp voneinander entfernt.

Es ist auch möglich, dass das Oligo mit einer Reporter-Markierung in einer niedrigeren Konzentration als das Oligo mit der Quencher-Markierung in der Reaktion vorliegt, vorzugsweise im Verhältnis 1:10 bis 1:20.

Das erfindungsgemäße Verfahren wird nachfolgend anhand von Ausführungsbeispielen erläutert, wobei die Ausführungsbeispiele keine Einschränkung des Verfahrens darstellen sollen.

**Ausführungsbeispiele**

Beispiel 1:

Nachweis der Influenza Typ H1N1 (swine origine cDNA) mittels in die PCR integriertem Hybridisierungsverfahren und Endpunkt- Fluoreszenzmessung einer Fluoreszenzlöschung

Im Ansatz werden negative Proben (NTC), H1N1 cDNA-positive Proben (POS) und die Proben, die zwar humanes DNA-Material (Tupferabstrich der Nasenschleimhaut), aber nicht H1N1 enthalten (NEG).

An diesem Beispiel wird die Möglichkeit einer erfindungsgemäßen Endpunktfluoreszenzmessung und die Spezifität der Methode demonstriert.

PCR- Primer /Sonde
H1 N1 sense Primer (5'-tgg gaa atc cag agt gtg aat cac tct c-3')
H1N1 antisense Primer (Oligo Typ 1) (5'-BHQ1-cgt tcc att gtc tga act agr tgt ttc c-3')
H1N1 Sonde (Oligo Typ 2) (5'- agc aag ctc atg gtc cta cat t-FAM- 3')

Proben: 3x POS; 3x NEG; 3x NTC

Pro Probe:

| | |
|---|---|
| sense Primer (25 pmol/$\mu$l) | 0,1 $\mu$l |
| antisense Primer (50 pmol/$\mu$l) | 0,1 $\mu$l |
| Sonde (5 pmol/$\mu$l) | 0,1 $\mu$l |
| dNTP- Mix (12,5 mM) | 0,3 $\mu$l |
| 10X PCR- Buffer (MgCl$_2$ included) | 1,5 $\mu$l |
| Taq- DNA- Polymerase | 0,75 U |
| PCR- Grade H$_2$O | add 15 $\mu$l |

Die PCR wird im SpeedCycler (Analytik Jena) unter der Nutzung der Rapid-Cycler- Technologie durchgeführt:

Amplifikations-/Hybridisierungskonditionen

| | |
|---|---|
| Schritt 1: Denaturierung | 98°C/90" |
| Schritt 2: Amplifizierung 41 Zyklen | ( 98°C/4"; 57°C /4"; 72°C /10" |
| Schritt 3: Denaturierung | 95°C/900" |
| Schritt 4: Hybridisierung | 43°C /600" |

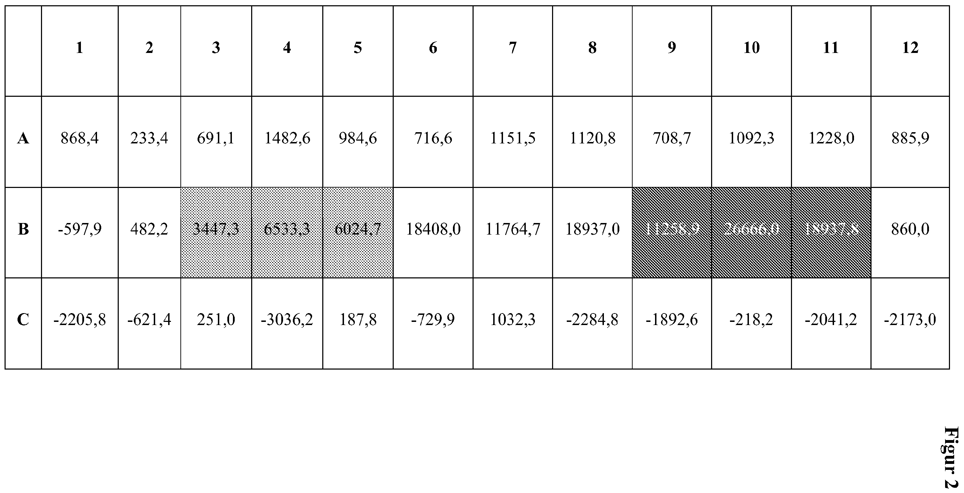

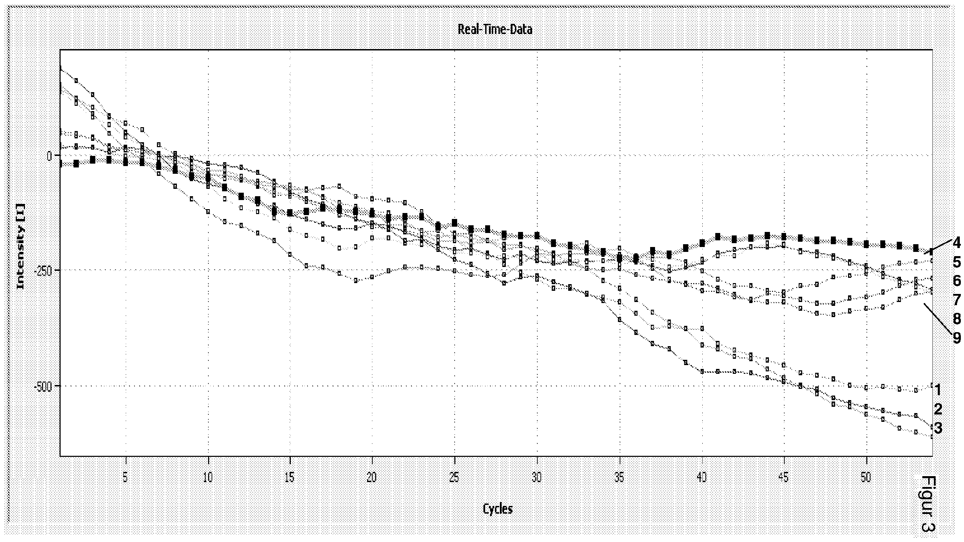

Der Nachweis des Amplifikationsereignisses/der Hybridisierungsreaktion erfolgt mittels einer Endpunktmessung mit dem Fluoreszenzreader SpeedScan (Analytik Jena AG; Figur 2). Gleichzeitig wurde die Veränderung der Fluoreszenzintensität der Probe während der gesamten Reaktion in Echtzeit (Figur 3) gemessen. Die Messdaten des SpeadScan-Geräts wurden einer Pos/Neg- Bestimmung nach folgender Formel unterzogen:

$$\bar{x}N - xN_{min} + \%\bar{x}N = A$$

$$\bar{x}N - P = B$$

$$wenn\ A - B \geq 0\ ist\ die\ Probe\ negativ$$

$$wenn\ A - B < 0\ ist\ die\ Probe\ positiv$$

Wo $\bar{x}N$ der Mittelwert der NTC- Werte; $xN_{min}$ der kleinste NTC- Wert; gewünschte Prozentabweichung des positiven Wertes von dem negativen (z.B. 20% vom $\overline{x}N$) und P- der Messwert der zu testenden Probe ist.

Weiterhin kann die Probe semiquantitativ ausgewertet werden, indem man einen Konzentrationsstandard benutzt.

Eine real quantitative Auswertung kann aber nur bei Vorhandensein einer internen Kontrolle und einer kompetitiven Reaktion erfolgen.

Die Ergebnisse der qualitativen Auswertung der Probenfluoreszenz sind in der Tabelle 1 zusammengefasst.

| Proben ID | Messwert nach der PCR | Wert A 20% | bei Wert B | A-B | pos/neg |
|---|---|---|---|---|---|
| Pos | 3447 | 11485 | 15506 | -4021 | POS |
| Pos | 6533 | 11485 | 12420 | -935 | POS |
| Pos | 6024 | 11485 | 12929 | -1444 | POS |
| Neg | 18408 | 11485 | 5573 | 5912 | NEG |
| Neg | 11764 | 11485 | 7189 | 4296 | NEG |
| Neg | 18937 | 11485 | 16 | 11469 | NEG |
| NTC | 11258 | 11485 | 7695 | 3790 | NEG |
| NTC | 26666 | 11485 | -7713 | 19198 | NEG |
| NTC | 18937 | 11485 | 16 | 11469 | NEG |

Beispiel 2:

Abhängigkeit der Signalintensität von der Konzentration der Target-DNA im Vergleich mit einer konventionellen Real-Time-PCR

Es wurden zwei Ansätze gemacht: für das erfindungsgemäße Verfahren und ein Real-Time-PCR-Ansatz mit einer FAM- BHQ1 markierten Sonde. Als Proben wurden die aus Influenza H1N1-Virusstämmen synthetisierte cDNA's (Partikelzahl/PCR-Ansatz s. Tabelle) verwendet.

Ansatz 1: Die Reaktionsbedingungen s. Beispiel 1

Ansatz 2:

PCR- Primer /Sonde
H1N1 RT sense Primer (5'- tgg gaa atc cag agt gtg aat cac t-c-3')
H1N1 RT antisense Primer (5'- cgt tcc att gtc tga act agr tgt t-3')
H1N1 RT Sonde (5'- **FAM**-cca caa tgt agg acc atg agc ttg ctg t-**BHQ1**- 3')

Pro Probe:

| | |
|---|---|
| sense Primer (50 pmol/$\mu$l) | 0,1 $\mu$l |
| antisense Primer (50 pmol/$\mu$l) | 0,1 $\mu$l |
| Sonde (25 pmol/$\mu$l) | 0,1 $\mu$l |
| dNTP- Mix (12,5 mM) | 0,3 $\mu$l |
| 10X PCR- Buffer (MgCl$_2$ included) | 1,5 $\mu$l |
| Taq- DNA- Polymerase | 0,75 U |
| .PCR- Grade H$_2$O | add 15 $\mu$l |

Die PCR wird im SpeedCycler (Analytik Jena) unter der Nutzung der Rapid-Cycler- Technologie durchgeführt:

Amplifikations-/Hybridisierungskonditionen
Schritt 1: Denaturierung    98°C/90"
Schritt 2: Amplifizierung    41 Zyklen (98°C/4"; 57°C/4"; 72°C/10"

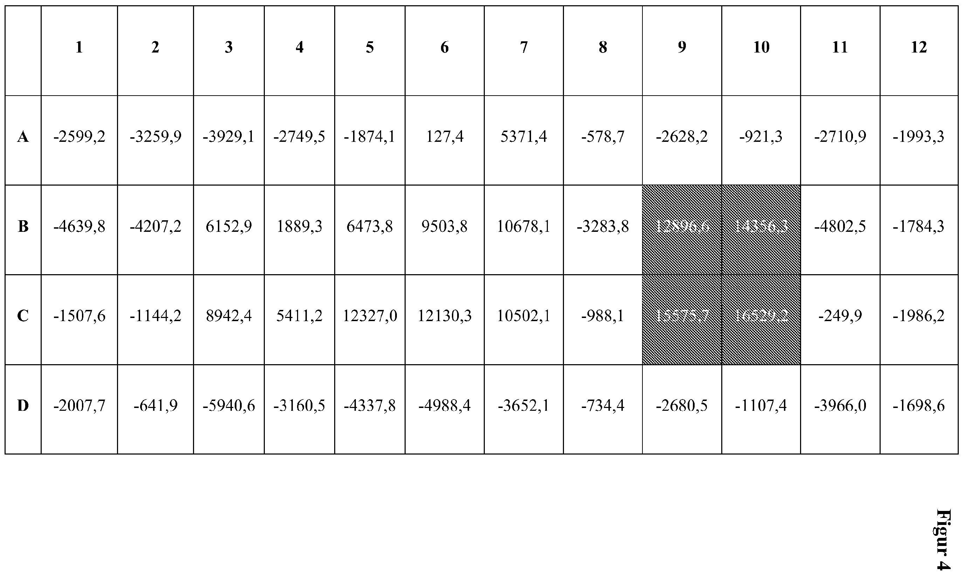

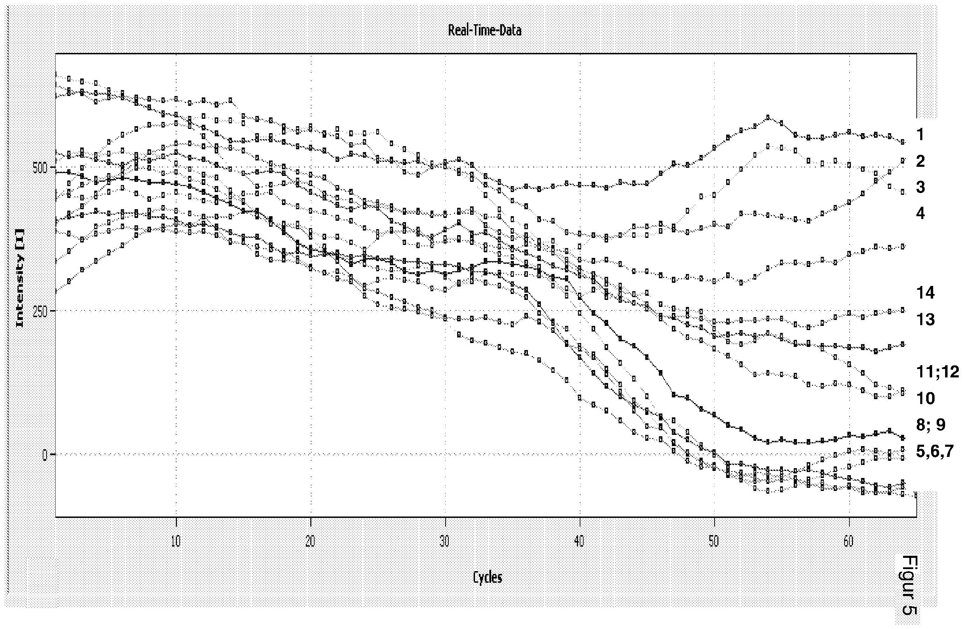

Der Nachweis des Amplifikationsereignisses/der Hybridisierungsreaktion bei Ansatz 1 erfolgte mittels einer Endpunktmessung der Fluoreszenz mittels SpeedScan (Analytik Jena AG; Figur 4). Gleichzeitig wurde die Veränderung der Fluoreszenzintensität der Probe während der gesamten Reaktionszeit in Echtzeit (RealTime-PCR) beobachtet (Figur 5). Die Messdaten des SpeadScan-Geräts wurden einer Pos/Neg-Bestimmung nach oben beschriebenen Formel (s. Beispiel 1) unterzogen. Bei Ansatz 2 wurde die Freisetzung der Fluoreszenz konventionell mittels RealTime-PCR gemessen.

Die Ergebnisse der qualitativen Auswertung der Probenfluoreszenz sind in der Tabelle 2 zusammengefasst.

| Partikelzahl in der Probe | Messwert nach der PCR (Mittelwert) | Wert A bei 20% | Wert B | A-B | pos/neg | Ct-Wert Ansatz 2 |
|---|---|---|---|---|---|---|
| 10000 | 7547 | 4910 | 7292 | -2382 | POS | 26 |
| 5000 | 3650 | 4910 | 11189 | -6279 | POS | 26 |
| 500 | 9400 | 4910 | 5439 | -529 | POS | 33 |
| 50 | 10816 | 4910 | 4023 | 887 | NEG | 38 |
| 5 | 10590 | 4910 | 4249 | 661 | NEG | NoCt |
| NTC | 14839 | 4910 | 0 | 4910 | NEG | NoCt |

**Erläuterung der Figuren**

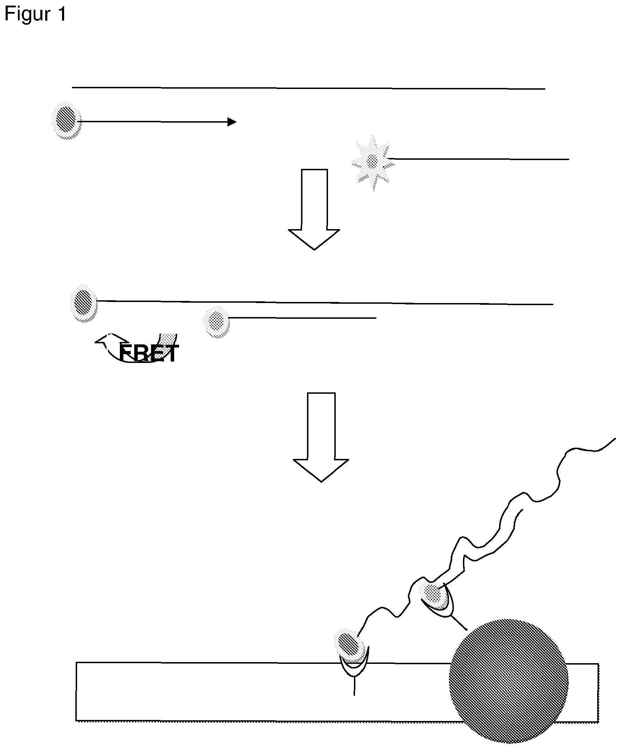

Figur 1 zeigt ein Schema des Verfahrensablaufs.

Figur 2 zeigt eine Fluoreszenzmessung nach dem Ablauf der Amplifizierungs-/ Hybridisierungs-Reaktion. Felder B3-B5 sind POS-Proben, B6-B8 sind NEG-Proben, B9-B11 sind NTC-Proben (Messung der Fluoreszenz mittels SpeedScan (Analytik jena AG)).

Figur 3 zeigt eine RealTime-Fluoreszenzmessung während der ganzen Amplifizierungs-/Hybridisierungs-Reaktion. Die Kurven 1-3 sind POS-Proben, 4-6 sind NEG-Proben, 7-9 sind NTC-Proben (Real Time Messung zur Nachverfolgung der Nachweisreaktion).

Figur 4 zeigt eine Fluoreszenzmessung nach dem Ablauf der Amplifizierungs-/ Hybridisierungs-Reaktion. Felder B3/C3 sind POS-Proben mit einem Viruspartikelanteil von 10000; B4/C4 - sind POS-Proben mit einem Viruspartikelanteil von 5000; B5/C5 - sind POS-Proben mit einem Viruspartikelanteil von 500; B6/C6 - sind POS-Proben mit einem Viruspartikelanteil von 50; B7/C7 - sind POS-Proben mit einem Viruspartikelanteil von 5; B9-B10 /C9-C10 sind NTC-Proben.

Figur 5 zeigt eine RealTime-Fluoreszenzmessung während der ganzen Amplifizierungs-/ Hybridisierungs- Reaktion. Die Kurven 1-4 sind NTC-Proben; Proben 5-14 sind wie folgt konzentriert: 5,6 - 100000 Partikel/Probe; 7, 8 - 5000 Partikel/Probe; 9, 10 500 Partikel/Probe; Probe 11, 12 - 50 Partikel/Probe; Probe 13, 14 - 5 Partikel/Probe.

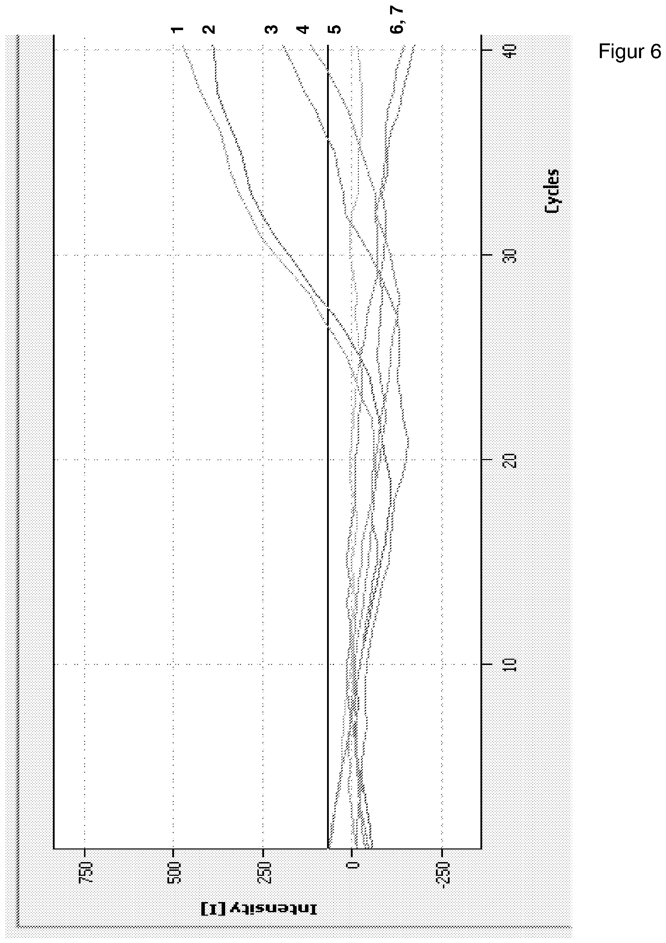

Figur 6 zeigt eine RealTime-Fluoreszenzmessung während einer RealTime-Reaktion mit einer konventionellen TaqMan-Sonde. Die Kurven 6,7 sind NTC-Proben; Proben 1-5 sind wie folgt konzentriert: 1 - 100000 Partikel/Probe; 2 - 5000 Partikel/Probe; 3 500 Partikel/Probe; Probe 4 - 50 Partikel/Probe; Probe 5 - 5 Partikel/Probe.

**Patentansprüche**

1. 1. Verfahren zum Nachweis spezifischer Nukleinsäuresequenzen (Target) mit folgenden Schritten:

   a) matrizenabhängige DNA-Neusynthese der Targetnukleinsäure,
   b) targetspezifische Sondenhybridisierung und
   c) Nachweis des Hybridisierungsereignisses,
   wobei dass im Schritt a) mindestens ein durch eine Markierung 1 markiertes Oligonukleotid 1, das vollständig oder teilweise zu der Targetsequenz komplementär ist, als Primer in der matrizenabhängigen Neusynthese der Targetnukleinsäure fungiert und im Schritt b) mindestens ein durch eine Markierung 2 markiertes Oligonukleotid 2, das beim Schritt a) zwar anwesend, aber aufgrund seiner niedrigeren Schmelztemperatur als das Oligonukleotid 1 nicht am Schritt a) beteiligt ist, aber mit dem DNA-Neusyntheseprodukt von Oligonukleotid 1 teilweise oder vollständig hybridisiert und zur targetspezifischen Sondenhybridisierung eingesetzt wird ,
   **dadurch gekennzeichnet, dass**.
   der Nachweis des Hybridisierungsereignisses in der Reaktionskavität erfolgt, in der die Amplifizierungs-/Hybridisierungsreaktion abläuft und dass die beiden Markierungen 1 und 2 als ein FRET-Paar fungieren, wobei der Nachweis in Folge des FRET-Effekts mittels einer Endpunktdetektion erfolgt.

2. Verfahren nach Anspruch 1, **dadurch gekennzeichnet, dass** als FRET-Paar vorzugsweise FITC/TAMRA, FAM/TAMRA oder FAM/BHQ1 dient und der Nachweis der in Folge eines FRET-Effekts auftretenden Fluoreszenzverminderung zu einer messbaren Verringerung der Fluoreszenz führt.

3. Verfahren nach Anspruch 1, **dadurch gekennzeichnet, dass** nach Schritt a) der Reaktionsansatz erhitzt bei einer Temperatur > 90°C wird, wobei es zur thermischen Strangtrennung kommt und anschließend der Reaktionsansatz auf die Hybridisierungstemperatur des Oligonukleotids 2 abgekühlt wird.

4. Verfahren nach einem der Ansprüche 1 bis 3, **dadurch gekennzeichnet, dass** während der DNA-Neusynthese / Hybridisierung das hybridisierte Oligonukleotid 2 nicht durch die Taq-Polymerase zerstört wird, sondern auch nach dem Reaktionsablauf im hybridisierten Zustand bleibt.

5. Verfahren nach einem der Ansprüche 1 bis 4, **dadurch gekennzeichnet, dass** der Nachweis des Hybridisierungsereignisses erst nach dem Abschluss der DNA-Neusynthese / Hybridisierung erfolgt.

6. Verfahren nach einem der Ansprüche 1 bis 5, **dadurch gekennzeichnet, dass** die Oligonukleotid 2 gegen die 5' → 3'-Polymeraseaktivität, vorzugsweise durch die Markierung selbst oder durch eine Phosphorylierung, geschützt ist.

**Claims**

1. A method for detecting specific nucleic acid sequences (target) comprising the steps of:

   a) matrix dependant DNA new synthesis of the target nucleic acid,
   b) target specific probe hybridization and
   c) detection of the hybridization event,
   wherein the oligonucleotide 1 marked in step a) at least by a marker 1, which is entirely or partially complementary to the target sequence, acts as a primer in the matrix dependent new synthesis of the target nucleic acid, and in step b) at least an oligonucleotide 2 marked by a marker 2, which is present in step a) but due to its lower melting temperature than the oligonucleotide 1 is not involved in step a) but partially or completely hybridizes with the DNA new synthesis product of oligonucleotide 1, and is used for target specific probe hybridization,
   **characterized in that**
   detection of the hybridization event occurs in the reaction cavity in which the amplification / hybridization reaction takes place, and that the two markers 1 and 2 act as a FRET pair, wherein detection occurs as a result of the FRET effect by means of an endpoint detection.

2. The method according to claim 1, **characterized in that** preferably FITC/TAMRA, FAM/TAMRA or FAM/BHQ1 serves as a FRET pair, and the detection of the fluorescence reduction occurring as a result of a FRET effect leads to a measurable reduction of fluorescence.

3. The method according to claim 1, **characterized in that** after step a) the reaction batch is heated at a temperature > 90°C, wherein thermal strand separation occurs, and subsequently the reaction batch is cooled to hybridization temperature of the oligonucleotide 2.

4. The method according to claim 1 to 3, **characterized in that** during the DNA new synthesis / hybridization, the hybridized oligonucleotide 2 is not destroyed by the Taq polymerase, but remains in the hybridized state also after the reaction has taken place.

5. The method according to any one of claims 1 to 4, **characterized in that** the detection of the hybridization event occurs only after completion of the DNA new synthesis / hybridization.

6. The method according to any one of claims 1 to 5, **characterized in that** the oligonucleotide 2 is protected against the 5' → 3' polymerase activity, preferably by the marker itself or by phosphorylation.

## Revendications

1. Procédé pour détecter des séquences d'acide nucléique spécifiques (cible) avec des étapes suivantes :

    a) matrice-dépendante nouvelle synthèse d'ADN de l'acide nucléique cible,
    b) hybridation de sonde spécifique à la cible et
    c) détection de l'événement d'hybridation,
    dans lequel l'oligonucléotide 1 marqué dans l'étape a) au moins par un marquage 1, qui est totalement ou partiellement complémentaire de la séquence cible, agit comme une amorce dans la matrice-dépendante synthèse nouvelle de l'acide nucléique cible, et dans l'étape b) au moins un oligonucléotide 2, marqué par un marquage 2, qui est présent dans l'étape a), mais ne participe pas à l'étape a) en raison de sa température de fusion plus basse que celle de l'oligonucléotide 1, mais s'hybride partiellement ou totalement avec le produit de la nouvelle synthèse d'ADN, et est utilisé pour l'hybridation de sonde spécifique à la cible,
    **caractérisé en ce que**
    la détection de l'événement d'hybridation a lieu dans la cavité de la réaction dans laquelle la réaction d'amplification / d'hybridation se déroule, et que les deux marquages 1 et 2 agissent comme une paire FRET, dans lequel la détection suivant l'effet FRET a lieu par l'intermédiaire d'une détection de point final.

2. Procédé selon la revendication 1, **caractérisé en ce que**, de préférence FITC/TAMRA, FAM/TAMRA ou FAM/BHQ1 sert comme une paire FRET, et que la détection de la réduction de la fluorescence se produisant à la suite d'un effet FRET mène à une réduction de la fluorescence mesurable.

3. Procédé selon la revendication 1, **caractérisé en ce qu'**après l'étape 1) la préparation de la réaction est chauffé à une température de > 90°C, où se produit la séparation thermique des brins, et ensuite la préparation de la réaction est refroidi à la température d'hybridation de l'oligonucléotide 2.

4. Procédé selon l'une quelconque des revendications 1 à 3, **caractérisé en ce que**, pendant la nouvelle synthèse d'ADN / l'hybridation, l'oligonucléotide hybridé 2 n'est pas détruit par la polymérase Taq, mais reste en état hybridé aussi après le processus de réaction.

5. Procédé selon l'une quelconque des revendications 1 à 4, **caractérisé en ce que** la détection de l'événement d'hybridation n'a lieu qu'après l'achèvement de la nouvelle synthèse d'ADN / l'hybridation.

6. Procédé selon l'une quelconque des revendications 1 à 5, **caractérisé en ce que** l'oligonucléotide 2 est protégé contre l'activité polymérase 5' → 3', de préférence, par le marquage il-même ou par une phosphorylation.

Figur 1

| | 1 | 2 | 3 | 4 | 5 | 6 | 7 | 8 | 9 | 10 | 11 | 12 |
|---|---|---|---|---|---|---|---|---|---|---|---|---|
| A | 868,4 | 233,4 | 691,1 | 1482,6 | 984,6 | 716,6 | 1151,5 | 1120,8 | 708,7 | 1092,3 | 1228,0 | 885,9 |
| B | -597,9 | 482,2 | 3447,3 | 6533,3 | 6024,7 | 18408,0 | 11764,7 | 18937,0 | 11255,9 | 26666,0 | 18917,8 | 860,0 |
| C | -2205,8 | -621,4 | 251,0 | -3036,2 | 187,8 | -729,9 | 1032,3 | -2284,8 | -1892,6 | -218,2 | -2041,2 | -2173,0 |

Figur 2

Figur 3

| | 1 | 2 | 3 | 4 | 5 | 6 | 7 | 8 | 9 | 10 | 11 | 12 |
|---|---|---|---|---|---|---|---|---|---|---|---|---|
| A | -2599,2 | -3259,9 | -3929,1 | -2749,5 | -1874,1 | 127,4 | 5371,4 | -578,7 | -2628,2 | -921,3 | -2710,9 | -1993,3 |
| B | -4639,8 | -4207,2 | 6152,9 | 1889,3 | 6473,8 | 9503,8 | 10678,1 | -3283,8 | (shaded) | (shaded) | -4802,5 | -1784,3 |
| C | -1507,6 | -1144,2 | 8942,4 | 5411,2 | 12327,0 | 12130,3 | 10502,1 | -988,1 | (shaded) | (shaded) | -249,9 | -1986,2 |
| D | -2007,7 | -641,9 | -5940,6 | -3160,5 | -4337,8 | -4988,4 | -3652,1 | -734,4 | -2680,5 | -1107,4 | -3966,0 | -1698,6 |

**Figur 4**

Figur 5

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5210015 A
- US 5487972 A
- JP 002001029072 A
- WO 2009126678 A2
- DE 10250948 A1
- DE 102005000021 A1
- EP 1384789 B1
- KR 1020060099022 A
- WO 2009000764 A2
- WO 200551967 A2
- WO 03072051 A2
- EP 0972848 A2
- EP 0826066 B1
- DE 102010003781

**In der Beschreibung aufgeführte Nicht-Patentliteratur**

- **BERNARD, P.S. et al.** *Anal Biochem,* 1998, vol. 255, 101-7
- **E LYON et al.** LightCycler Technology in Molecular Diagnostics. *J. Mol. Diagn,* 2009, vol. 11 (2), 93-101
- **PIEPENBURG et al.** *PLOS biologie,* Juli 2006, vol. 4 (7), 1115-1121

SEQUENCE LISTING

<110> AJ INNUSCREEN GMBH GRASER, Elmara HILLEBRAND, Timo

<120> VERFAHREN ZUM NACHWEIS SPEZIFISCHER NUKLEINSÄURESEQUENZEN

<130> 1120

<140> unknown
<141> 2011-04-08

<150> DE 10 2010 003 781.8
<151> 2010-04-08

<160> 6

<170> PatentIn version 3.3

<210> 1
<211> 28
<212> DNA
<213> H1N1 sense Primer

<400> 1
tgggaaatcc agagtgtgaa tcactctc 28

<210> 2
<211> 28
<212> DNA
<213> H1N1 antisense Primer

<400> 2
cgttccattg tctgaactag rtgtttcc 28

<210> 3
<211> 22
<212> DNA
<213> H1N1 probe

<400> 3
agcaagctca tggtcctaca tt 22

<210> 4
<211> 26
<212> DNA
<213> H1N1 RT sense Primer

<400> 4
tgggaaatcc agagtgtgaa tcactc 26

<210> 5
<211> 25
<212> DNA
<213> H1N1 RT antisense Primer

<400> 5
cgttccattg tctgaactag rtgtt 25

<210> 6
<211> 28
<212> DNA
<213> H1N1 RT probe

<400> 6
ccacaatgta ggaccatgag cttgctgt 28